# FASCICULE DE BREVET EUROPEEN

(11) **EP 0 549 387 B1**
(45) Date de publication et mention de la délivrance du brevet: **27.09.1995**
(21) Numéro de dépôt: 92403205.5
(22) Date de dépôt: 27.11.1992
(51) Int. Cl.: C07C 17/10, C07C 19/08

(54) **Synthèse du bromure de n.perfluorooctyle**
Synthese von n-Perfluoroctylbromid
Synthesis of n-perfluorooctylbromide

(30) Priorité: 20.12.1991 FR 9115877
(43) Date de publication de la demande: 30.06.1993
(73) Titulaire: ELF ATOCHEM S.A., 92800 Puteaux (FR)
(72) Inventeur: Drivon, Gilles, F-69850 Saint Martin en Haut (FR); Kervennal, Jacques, F-69005 Lyon (FR)
(74) Mandataire: Leboulenger, Jean

(56) Documents cités:
- EP-A- 0 428 039
- EP-A- 0 429 331
- FR-A- 1 512 068

## Description

La présente invention concerne le domaine des bromures de perfluoroalkyle et a plus particulièrement pour objet la préparation du bromure de n.perfluorooctyle C₈F₁₇Br.

Ce composé, également connu sous l'abréviation PFOB, est utilisé dans de nombreux domaines, en particulier en médecine comme radiopaque (agent de contraste aux rayons X) ou comme transporteur d'oxygène dans les substituts du sang.

Dans son brevet EP 0 298 870 et son brevet EP 0 429 331 la Demanderesse a décrit des procédés de fabrication des bromures de perfluoroalkyle R_{F}Br (en particulier du PFOB) à partir des chlorures de perfluoroalcanesulfonyle R_{F}SO₂Cl correspondants qu'on fait réagir soit avec HBr gazeux en présence d'un catalyseur (EP 0 298 870), soit avec un bromure d'ammonium ou de phosphonium quaternaire (EP 0 429 331). Les rendements obtenus sont généralement élevés, mais le sulfochlorure R_{F}SO₂Cl utilisé est une matière première déjà élaborée puisque sa synthèse à partir de l'iodure correspondant R_{F}I nécessite deux étapes réactionnelles suivant l'équation : 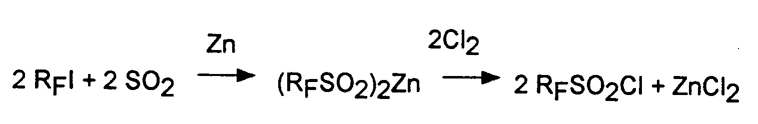
Une autre voie d'obtention des composés R_{F}Br consiste en la bromation thermique des iodures correspondants R_{F}I, ces derniers étant des produits disponibles en quantités industrielles. Bien que cette voie soit la plus directe, elle présente l'inconvénient de conduire à un produit contenant toujours du R_{F}I résiduel qu'il est difficile de séparer du R_{F}Br désiré. Ceci est plus particulièrement le cas du PFOB qui doit avoir une teneur en C₈F₁₇I inférieure à 100 ppm pour ses applications médicales.

Une technique connue depuis longtemps et exploitée industriellement pour la fabrication du bromure de trifluorométhyle CF₃Br consiste en la bromation thermique du trifluorométhane. En l'absence de catalyseur, cette réaction est généralement conduite à des températures allant de 600 à 700°C de manière a' obtenir un taux de transformation élevé.

Le brevet US 3 456 024 concerne un procédé de préparation de chlorures ou bromures de perfluoroalkyle ou de perfluoroalkylène qui consiste à faire réagir, dans un domaine de températures allant de 450 à 700°C, du chlore ou du brome avec divers composés comportant un radical perfluoroalkyle R_{f} ou perfluoroalkylène R'_{f} tels que les composés R_{f}SF₅, R_{f}SO₂X, (R_{f}SO₂)₂O, HR'_{f}CH₂OH, R_{f}H et R_{f}(CH₂CH₂)ₙX', X désignant un atome de fluor, chlore ou brome ou un groupe hydroxy, X' un atome de chlore ou de brome, et n un nombre allant de 1 à 5. Dans ce brevet, la bromation d'un hydrogéno-perfluoroalcane R_{f}H n'est illustrée que par un seul exemple, à savoir l'exemple 7 relatif à la bromation du composé n.C₇F₁₅H avec les rendements molaires en C₇F₁₅Br suivants :

| **Température (°C)** | **Rendement** |
|---|---|
| 500 | 29 % |
| 550 | 49,5 % |
| 600 | 81,4 % |

Il n'est fait aucune mention de la sélectivité de la réaction, paramètre très important pour la mise en oeuvre d'un procédé industriel.

Considérant les températures élevées nécessaires pour obtenir des taux de transformation industriellement acceptables tant dans la bromation du trifluorométhane que dans celle du composé n-C₇F₁₅H, l'application de cette technique à la préparation industrielle du PFOB pour usage médical à partir de l'hydrogéno-1 heptadécafluoro n.octane (C₈F₁₇H) ne pouvait pas être sérieusement envisagée. En effet, ce composé est thermiquement instable et se décompose à partir de 510°C en produits toxiques, notamment en perfluoroisobutylène (PFIB) connu pour son extrême toxicité (voir par exemple les articles de E.W. Cook et J.S. Pierce, Nature, 242, 1973, p. 5396-7 et de J.W. Clayton, Environmental Health Perspectives, 21, 1977, pp. 255-267). D'autre part, le PFOB lui-même est thermiquement instable à partir de 520°C.

Il a maintenant été trouvé que, dans le cas particulier du composé C₈F₁₇H et de son dérivé bromé, on peut obtenir un rendement élevé de la réaction tout en opérant à des températures plus faibles que celles enseignées par la technique antérieure.

Le procédé selon l'invention pour la préparation continue du PFOB est caractérisé en ce que l'on fait réagir en phase gazeuse du brome et de l'hydrogéno-1 heptadécafluoro n.octane dans un rapport molaire Br₂/C₈F₁₇H compris entre 0,2 et 5 (de préférence entre 0,5 et 2,5), à une température comprise entre 450 et 520°C (de préférence entre 470 et 510°C) et avec un temps de contact compris entre 2 et 240 secondes (de préférence entre 5 et 60 secondes).

Cet ensemble de conditions opératoires conduit à un taux de transformation élevé et à une sélectivité au moins égale à 99 %, sans formation de sous-produits toxiques comme le PFIB. Le PFOB brut obtenu est en outre exempt de toute trace (limite de détection : ≦100 ppm) de C₈F₁₇I résiduel éventuellement contenu dans le C₈F₁₇H de départ.

Le procédé selon l'invention peut être mis en oeuvre dans tout type de réacteur adapté à une réaction en phase gazeuse, en particulier dans un réacteur tubulaire éventuellement muni d'un garnissage pour favoriser le mélange des gaz. On utilise avantageusement un appareillage en quartz ou en verte, mais on peut également employer tout matériau métallique (par exemple Monel, Inconel, Hastelloy) capable de résister à l'action corrosive du brome et de l'acide bromhydrique à la température réactionnelle choisie.

Bien que cela ne soit pas indispensable, la réaction du brome et de C₈F₁₇H peut être effectuée en présence d'un diluant gazeux inerte tel que, par exemple, l'azote ou le PFOB lui-même.

Industriellement, on préfère travailler à la pression atmosphérique, mais on ne sortirait pas du cadre de la présente invention en travaillant à une pression supérieure à la pression atmosphérique, pourvu que le système réactionnel reste à l'état gazeux.

Les gaz sortant du réacteur qui contiennent le PFOB formé et le C₈F₁₇H non transformé, ainsi que l'acide bromhydrique sous-produit par la réaction :

C₈F₁₇H+Br₂→C₈F₁₇Br+HBr

et éventuellement du brome non réagi ou en excès sont refroidis, puis neutralisés par une solution aqueuse alcaline et/ou réduits par une solution aqueuse de sulfite ou métabisulfite de sodium. Après décantation de la phase organique, le composé C₈F₁₇H non transformé peut, après séparation par distillation, être recyclé à l'entrée du réacteur.

Conformément à une modalité avantageuse, les gaz sortant du réacteur peuvent, avant neutralisation et/ou réduction, être traités par du chlore de manière à réoxyder l'acide bromhydrique sous-produit en brome qui peut ainsi être recyclé dans le procédé.

Les exemples suivants illustrent l'invention sans la limiter. 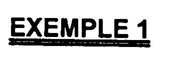
On utilise un réacteur tubulaire en quartz (diamètre intérieur : 60 mm; hauteur : 700 mm) chauffé uniformément sur toute sa hauteur et garni au fond sur une hauteur de 150 mm d'anneaux de quartz (diamètre : 5 mm) afin de favoriser le mélange des gaz.

L'entrée inférieure du réacteur est précédée d'un évaporateur dans lequel on introduit simultanément et en continu du brome à un débit de 320 g/h et C₈F₁₇H à un débit de 420 g/h, soit un rapport molaire Br₂/C₈F₁₇H égal à 2/1 à l'entrée du réacteur.

La température réactionnelle est fixée à 500°C et le temps de contact des réactifs à 60 secondes.

En sortie du réacteur, les produits sont refroidis, réduits par une solution de sulfite de sodium, puis lavés par une solution légèrement alcaline. L'analyse chromatographique en phase gazeuse indique un taux de transformation globale (TT_{G}) du C₈F₁₇H égal à 83% et une sélectivité en C₈F₁₇Br de 100%. 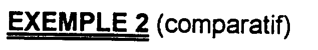
On opère comme à l'exemple 1 mais à 550°C au lieu de 500°C.

Le taux de transformation global de C₈F₁₇H est plus élevé (90,5 %), mais on observe un chute très importante de la sélectivité en C₈F₁₇Br (82 %). 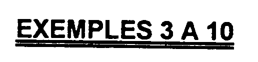
On opère comme à l'exemple 1 mais avec différents réglages des paramètres de fonctionnement (température réactionnelle, rapport molaire Br₂/C₈F₁₇H, temps de contact).

Les conditions opératoires et les résultats obtenus sont rassemblés dans le tableau suivant.

| **EXEMPLE** | **CONDITIONS OPERATOIRES** | | | **RESULTATS** | |
|---|---|---|---|---|---|
| | Température (°C) | Rapport molaire Br₂/C₈F₁₇H | Temps de contact (s) | TTG (%) du C₈F₁₇H | Sélectivité en C₈F₁₇Br |
| 3 | 450 | 1 | 30 | 21 | 100 |
| 4 | 500 | 1,02 | 30 | 59 | 99,8 |
| 5* | 550 | 0,99 | 30 | 72,5 | 87,5 |
| 6 | 500 | 2 | 30 | 71,5 | 99,7 |
| 7* | 550 | 2,03 | 30 | 90 | 92,3 |
| 8 | 500 | 0,96 | 60 | 66,5 | 99,1 |
| 9* | 550 | 1 | 60 | 74,5 | 78 |
| 10* | 525 | 1,5 | 45 | 85 | 97,0 |

| | | | | | |
|---|---|---|---|---|---|
| *Exemple comparatif | | | | | |

On constate systématiquement une chute brutale de la sélectivité lorsqu'on passe de 500 à 550°C, quel que soit le rapport molaire et/ou le temps de contact utilisé. Cette diminution de la sélectivité est sensible dès 525°C (exemple 10*) ce qui confirme bien I'instabilité thermique du C₈F₁₇H et du C₈F₁₇Br dès 520°C.

## Revendications

1. Procédé continu de préparation du bromure de n.perfluorooctyle, caractérisé en ce que l'on fait réagir en phase gazeuse du brome et de l'hydrogéno-1 heptadécafluoro n.octane C₈F₁₇H dans un rapport molaire Br₂/C₈F₁₇H compris entre 0,2 et 5, à une température comprise entre 450 et 520°C, et avec un temps de contact compris entre 2 et 240 secondes.

2. Procédé selon la revendication 1, dans lequel on opère à une température comprise entre 470 et 510°C.

3. Procédé selon la revendication 1 ou 2, dans lequel le rapport molaire Br₂/C₈F₁₇H est compris entre 0,5 et 2,5.

4. Procédé selon l'une des revendications 1 à 3, dans lequel le temps de contact est compris entre environ 5 et 60 secondes.

5. Procédé selon l'une des revendications 1 à 4, dans lequel on opère en présence d'un diluant inerte.

6. Procédé selon l'une des revendications 1 à 5, dans lequel on opère à la pression atmosphérique.

## Claims

1. Continuous process for preparation of n-perfluorooctyl bromide, characterised in that bromine is reacted in the gaseous phase with 1-hydroheptadecafluoro-n-octane C₈F₁₇H in a Br₂/C₈F₁₇H mole ratio between 0.2 and 5, at a temperature between 450 and 520°C, and with a contact time between 2 and 240 seconds.

2. Process according to Claim 1, in which the procedure is carried out at a temperature between 470 and 510°C.

3. Process according to Claim 1 or 2, in which the Br₂/C₈F₁₇H mole ratio is between 0.5 and 2.5.

4. Process according to one of Claims 1 to 3, in which the contact time is between approximately 5 and 60 seconds.

5. Process according to one of Claims 1 to 4, in which the procedure is carried out in the presence of an inert diluent.

6. Process according to one of Claims 1 to 5, in which the procedure is carried out at atmospheric pressure.

## Patentansprüche

1. Kontinuierliches Verfahren zur Herstellung von n-Perfluoroctylbromid, dadurch gekennzeichnet, daß man in gasförmiger Phase Brom und 1-Hydrogenoheptadecafluoro-n-octan, C₈F₁₇H in einem Br₂/C₈F₁₇H-Molverhältnis zwischen 0,2 und 5, bei einer Temperatur zwischen 450 und 520 °C und mit einer Kontaktzeit zwischen 2 und 240 Sekunden reagieren läßt.

2. Verfahren nach Anspruch 1, wobei man bei einer Temperatur zwischen 470 und 510 °C verfährt.

3. Verfahren nach Anspruch 1 oder 2, wobei das Br₂/C₈F₁₇H-Verhältnis zwischen 0,5 und 2,5 liegt.

4. Verfahren nach einem der Ansprüche 1 bis 3, wobei die Kontaktzeit zwischen ungefähr 5 und 60 Sekunden liegt.

5. Verfahren nach einem der Ansprüche 1 bis 4, wobei man in Gegenwart eines inerten Verdünnungsmittels verfährt.

6. Verfahren nach einem der Ansprüche 1 bis 5, wobei man unter atmosphärischem Druck verfährt.
